Europäisches Patentamt

(19) European Patent Office

Office européen des brevets

(11) Publication number: **0 163 573**
**B1**

(12) **EUROPEAN PATENT SPECIFICATION**

(45) Date of publication of patent specification: 06.09.89

(51) Int. Cl.⁴: **C 12 N 15/00**, C 12 N 9/50, C 12 P 19/34, C 12 P 21/00, C 07 K 7/08

(21) Application number: 85400985.9

(22) Date of filing: 20.05.85

(54) Site-specific proteolysis by renin.

(30) Priority: 24.05.84 US 614085

(43) Date of publication of application:
04.12.85 Bulletin 85/49

(45) Publication of the grant of the patent:
06.09.89 Bulletin 89/36

(84) Designated Contracting States:
AT BE CH DE FR GB IT LI LU NL

(56) References cited:
EP-A-0 089 626

Klinische Pothophysiologie (1973), Georg
Thierne Verlag, Stuttgart, 2. Auflage, Seite 575
Blundell, T. et al. (1983), Nature, vol. 304, pp
273-275

Klassische und molekulare Genetik (1972), C.
Bresch und R. Hausmann, Springer Verlag,
Seite 254

(73) Proprietor: MERCK & CO. INC.
126, East Lincoln Avenue P.O. Box 2000
Rahway New Jersey 07065-0900 (US)

(72) Inventor: Boger, Joshua S.
7.19 New Gulph Road
Bryn Mawr Pennsylvania 19010 (US)
Inventor: Haffey, Mary L.
901 Cross Lane
Blue Bell Pennsylvania 19422 (US)
Inventor: Scolnick, Edward M.
811 Wicksfield Road
Wynnewood Pennsylvania 19096 (US)

(74) Representative: Warcoin, Jacques et al
Cabinet Régimbeau 26, avenue Kléber
F-75116 Paris (FR)

Courier Press, Leamington Spa, England.

**Description**

Background of the invention

The use of recombinant DNA technology has permitted the expression of foreign proteins in both procaryotic and eucaryotic hosts. However, foreign proteins cannot always be expressed stably and to high levels in any given host cell. One way in which this problem has been circumvented is the production of fusion or hybrid proteins in which the protein of interest is covalently attached to a protein which is stably expressed in the chosen host. In many cases these fusion proteins are stable and are expressed at high levels. Identification and purification of the protein of interest is often facilitated by following the activity of the stably expressed component of the fusion protein (e.g., β - galactosidase).

A major disadvantage of the fusion protein systems is the difficulty often encountered in separating the protein of interest from the stably expressed protein which may be undesired in the final product.

In some cases, the protein of interest lacks sites for chemical (e.g., cyanogen bromide) or proteolytic (e.g., trypsin) cleavage while the stably expressed protein contains these cleavage sites. Then the fusion protein can be cleaved to yield the protein of interest as a distinct molecule containing little or none of the stably expressed protein.

However, treatment of fusion proteins by such general methods can result in degradation of the protein of interest as well as the stably expressed protein.

Objects of the invention

It is, accordingly, an object of the present invention to provide a unique oligonucleotide which codes for an amino acid sequence recognized by a highly specific proteolytic agent, such as renin. Another object is to provide a stretch of DNA containing this oligonucleotide between the nucleic acid sequences coding for the protein of interest and the nucleic acid sequences coding for the stably expressed protein. A further object is to provide a fused protein which contains between the protein of interest and the stably expressed protein a unique amino acid sequence recognized by a highly specific proteolytic agent. These and other objects of the invention will be apparent from the following description.

Summary of the invention

An oligonucleotide which codes for an amino acid sequence recognized and cleaved by the enzyme, renin, has been inserted into a plasmid expression vector. This nucleotide sequence was inserted within a sequence of DNA coding for a fusion protein comprised of two distinct domains and was inserted at the precise junction of these two domains. This new construction produces a novel protein which can be specifically cleaved by renin to yield two distinct molecules corresponding to the two domains of the original fusion protein.

Detailed description of the invention

The highly specific endopeptidase, renin, functions in nature to generate angiotensin I from the pro-hormone, angiotensin. In plasma, the formation of angiotensinigen I by renin constitutes the primary and rate-limiting step in a series of reactions which result in a rapid elevation of blood pressure and increased renal sodium retention. Renin exhibits extremely restricted substrate specificity and shows little, if any, general protease activity. Several synthetic peptides, the smallest of which are heptapeptides, also act as a substrate for renin.

It would be extremely beneficial therefore to introduce an oligonucleotide coding for such a unique protein sequence recognized by a highly specific proteolytic enzyme such as renin into a gene coding for a fusion protein between the nucleotides coding for the protein of interest and those coding for the stably expressed protein. Then, when the fusion protein is expressed, the highly specific proteolytic enzyme will cleave the fusion protein to yield the protein of interest as an intact protein product.

Renin, which is most readily obtained from submaxillary glands of mice, but which can be obtained from other mammalian sources including human, cleaves the following amino acid sequence:

| 7 | 8 | 9 | 10 | 11 | 12 | 13 |
|-----|-----|-----|-----|-----|-----|-----|
| Pro | Phe | His | Leu | Leu | Val | Tyr |

while human renin also cleaves the amino acid sequence:

Pro Phe His Leu Val Ile His

Preferably, the N-terminal amino group of proline is coupled to another amino acid, e.g., histidine. Various substitutions may be in the foregoing heptapeptide as shown by the following table:

TABLE I

| Heptapeptide amino acid | Permissible substituent |
|---|---|
| Phe | Trp, Tyr, Leu |
| His | Leu, Tyr, Ala, Ser, Lys |
| Leu (10) | Phe |
| Leu (11) | Ala |
| Val | Ile, Leu, Ala |
| Tyr | Phe, Trp, Leu |

If desired, longer amino acid sequences may be employed such as, for example, the following:

TABLE II

```
                        Pro Phe His Leu Leu Val Tyr Ser
                    His Pro Phe His Leu Leu Val Tyr
                    His Pro Phe His Leu Leu Val Tyr Ser
                Ile His Pro Phe His Leu Leu Val Tyr
                Ile His Pro Phe His Leu Leu Val Tyr Ser
            Tyr Ile His Pro Phe His Leu Leu Val Tyr
            Tyr Ile His Pro Phe His Leu Leu Val Tyr Ser
        Val Tyr Ile His Pro Phe His Leu Leu Val Tyr
        Val Tyr Ile His Pro Phe His Leu Leu Val Tyr Ser
    Arg Val Tyr Ile His Pro Phe His Leu Leu Val Tyr
    Arg Val Tyr Ile His Pro Phe His Leu Leu Val Tyr Ser
Asn Arg Val Tyr Ile His Pro Phe His Leu Leu Val Tyr
Asn Arg Val Tyr Ile His Pro Phe His Leu Leu Val Tyr Ser
        (Pro)ₙ His Pro Phe His Leu Leu Val Tyr
        (Pro)ₙ His Pro Phe His Leu Leu Val Tyr Ser
```

wherein n is an integer of from 1 to 5.

In the case of human renin, longer amino acid sequences also may be employed such as for example, the following:

TABLE III

```
            Pro Phe His Leu Val Ile His Ser
        His Pro Phe His Leu Val Ile His
    Ile His Pro Phe His Leu Val Ile His
    Ile His Pro Phe His Leu Val Ile His Ser
(Pro)ₙ His Pro Phe His Leu Val Ile His
(Pro)ₙ His Pro Phe His Leu Val Ile His Ser
```

where n is an integer of from 1 to 5.

Substituents indicated in the Table I also may be made in the corresponding sequences of the peptides of Tables II and III. Other substitutions are possible in the amino acids to the left of the proline in 7-position.

In carrying out the present invention it is first necessary to prepare a DNA oligonucleotide which, as a minimum, codes for the amino acid sequence Pro Phe His Leu Leu Val Tyr or a permissible equivalent as indicated above, or a longer amino acid sequence with these permissible equivalents as indicated above.

The nucleotide sequence which codes for the protein of interest is inserted into a suitable expression vector in an orientation suitable for expression of the protein of interest as a fusion protein. The vector is opened at a preselected site by means of a suitable restriction endonuclease and the vector is ligated with the hybridized oligonucleotide linker of Example 1 using a suitable DNA ligase. The ligated vector is then inserted into a prokaryotic or eukaryotic host for expression of the fusion protein. The expressed fusion protein is then treated with renin to cleave the protein of interest from the rest of the fusion protein. Alternatively, the linker can be coupled directly to the gene coding for the protein of interest and the resulting DNA construct can be inserted into a suitable expression vector in such manner that the protein of interest and DNA construct are expressed as a fusion protein.

While the following examples illustrate the present invention with respect to a particular fusion protein containing an immunogenic fragment from Epstein-Barr virus (EBV), it is to be understood that the present invention is applicable to any protein of interest expressed from a transformed host as a fusion protein. Examples of other proteins include viral antigens, bacterial antigens, protozoan antigens, rickettsial antigens, helminthic antigens, peptide and polypeptide hormones, growth factors, enzymes, oncogenic proteins, and other biologically active peptides.

An example of a specific DNA oligonucleotide which codes for the minimal amino acid sequence cleaved by renin of human or non-human origin is

CCG TTC CAC CTG CTC GTC TAC

while a specific example of a DNA oligonucleotide which codes for the minimal amino acid sequence cleaved by renin of human origin is

CCG TTC CAC CTG GTC ATA CAC

In each case it is preferable, although not essentially, to have an additional codon at the 5' end which codes for His, e.g., CAC.

The following examples illustrate the present invention without, however, limiting the same thereto.

Example 1
Synthesis of synthetic oligonucleotide linker
The following 32 base complementary oligonucleotides:

1.    5' CA CAC CCG TTC CAC CTG CTC GTC TAC CTT AAC 3'

2.    5' GT TAA GGT AGA CGA GCA GGT GGA ACG GGT GTG 3'

were synthesized separately on an Applied Biosystems, Inc. model No. 380A oligonucleotide synthesizer using phosphoramidite chemistry then deprotected and purified through Sephadex® G50 column chromatography as described by the manufacturer. Oligonucleotide 1 contained six extra nucleotides at the 3' end so that its complementary strand, nucleotide 2, would contain a stop signal in the correct reading frame if the DNA were inserted in the wrong orientation. The fraction eluting at 40% of the bed volume of the Sephadex® column was retained. The eluting buffer, triethylammonium bicarbonate (TEAB), 0.1 M, was removed by three consecutive cycles of lyophilization and resuspension in distilled water. The samples were resuspended in water to a concentration of 1 mg/ml. 10 microliters of each synthetic oligonucleotide were mixed in a test tube, heated to 65°C for 15 minutes and cooled at room temperature for 90 minutes to yield the double stranded oligonucleotide.

Example 2
Construction of the oligonucleotide containing vector
A part of the EBV membrane antigen (MA) gene was inserted into the β-galactoside expression vector pMC 1511 at the SmaI site by a procedure analogous to that described by Hennesy et al., PNAS 80: 5665—5669, 1983. The EBV DNA used to make this construction was a PstI fragment approximately 1,900 base pairs in length contained within the BamHIL fragment of EBV DNA, strain B95-8, which had been treated with Bal-31 nuclease to remove approximately 160 bases at each end. The EBV fragment begins with the sequence 5'-CATTCAGTCCAA-3' and ends with the sequence 5'-TTACGGCGGTGATTCAA-3'.

Plasmid DNA from the EBV-MA-β-gal expression vector described above was purified according to the procedure of Meagher et al., Cell 10:521—536, 1977 and further purified by centrifugation in a CsCl equilibrium density gradient (Maniatis et al., Molecular Cloning, A Laboratory Manual, Cold Spring Harbor Press, Cold Spring Harbor, New York, 1982, pp. 93—99). This and all of the following reactions were performed in 1.5 ml microfuge tubes. Plasmid DNA was digested with the restriction endonuclease BamHI (New England Biolabs, Beverly, Massachusetts) for 2 hours at 37°C using approximately 0.5 units of enzyme per microgram of DNA in a buffer containing 10 mM Tris-HCl pH 7.4, 50 mM NaCl, 10 mM MgSO$_4$, 1 mM dithiothreitol. The DNA was extracted using an equal volume of phenol:chloroform (1:1), the aqueous phase removed and re-extracted with an equal volume of chloroform. The aqueous phase was removed and adjusted to 0.3 M sodium acetate pH 7.0. The sample was diluted with 2.5 volumes of anhydrous ethanol and incubated on dry ice for 15 minutes to precipitate the DNA. The DNA was pelleted at 12K×g in a microfuge for 5 minutes, the pellet was rinsed with 70% ethanol and dried under vacuum. The dry pellet was dissolved in deionized, distilled water and the overhanging 5' ends were filled in using the Klenow fragment of DNA polymerase I. The reaction mixture contained 57 mM Tris-HCl pH 7.8, 12 mM MgCl$_2$, 50 mM NaCl, 10 mM β-mercaptoethanol, 10 micrograms/ml nuclease-free bovine serum albumin (BSA), 0.15 mM of each of the following deoxynucleotides: dATP, dCTP, dGTP and dTTP, and approximately 0.1 units of the Klenow fragment of DNA polymerase I (Boehringer Mannheim, Indianapolis, Indiana) per microprogram of DNA. The mixture was incubated at room temperature (approximately 20°C) for 20 minutes. The DNA was extracted and precipitated as described above. The vacuum dried DNA pellet was dissolved in deionized, distilled water. A ligation mixture was set up containing approximately 4 micrograms of the above treated DNA, 7.5 micrograms of the hybridized oligonucleotide described in Example 1, 0.1 mM ATP, 400 units T4 DNA ligase (new England Biolabs), 50 mM Tris-HCl pH 7.5, 10 mM MgCl$_2$, 10 mM dithiothreitol, 1 mM spermidine, and 0.1 microgram/ml of nuclease free bovine serum albumin (BSA). The reaction mixture was incubated at room temperature (approximately 20°C) for 3 hours.

Example 3

Transformation of cells with vector and expression of new sequence

Frozen competent *E. coli* strain AMA 1004 (lac Y$^+$, lac Z$^-$) were prepared according to the procedure of Dager *et al.* Gene *6*:23—28, 1979. Aliquots of 200 microliters were stored at −70°C until used. A single aliquot was thawed on ice, half of the ligation mixture described in Example 2 was added to the cells on ice and incubated for 1 hour. At that time the tube containing the transformation mixture was placed in a 45°C water bath for 90 seconds, then placed at room temperature for 5 minutes. L-Broth (Bacto tryptone 10 g, Bacto yeast extract 5 g, and NaCl 5 g, brought up to 1 l with distilled water), 1 ml, was added to the tube and the mixture was incubated at 37°C for 30 minutes. The transformation mixture was divided and plated onto 4 MacConkey agar plates containing 100 micrograms/ml of ampicillin. The plates were incubated at 37°C overnight in a hot air incubator. Approximately 5—10 light red (β-Gal$^-$) and approximately 20—30 dark red (β-Gal$^+$) clonies grew on each plate. The bacterial colonies were screened for the presence of the oligonucleotide sequence according to the procedure described in Maniatis *et al., supra*, pages 313—314. Briefly, 104 random colonies were picked onto master plates and nitrocellulose filters and grown at 37°C for 4 hours. Then the filters were treated according to procedure I of Maniatis *et al., supra.* to lyse the bacteria and bind liberated DNA, with the exception that 20 X SSC (3M sodium chloride, 0.3M sodium citrate) was used instead of 20 X SSPE.

One microgram of the oligonucleotide No. 1 described in Example 1 was labeled using bacteriophage T4 polynucleotide kinase (12 units, lot 35, New England Biolabs) and [γ-$^{32}$P]-ATP (adenosine triphosphate, 0.3 mCi; 3000 Ci/mM, Amersham Corp., Arlington Heights, Illinois) in a total volume of 30 microliters (Maniatis *et al., supra*, p. 122). The labeled probe was separated from unincorporated ATP by chromatography on C-18 resin (55—105 micron microbondapak, Waters/Millipore Corp.) as follows: an RT-20 microliter pipette tip (Rainin Corp., Woburn, Massachusetts) was plugged with siliconized glass wool (Maniatis *et al., supra*, p. 437). Atop the plug was added a few milligrams of C-18 resin. Another plug of glass wool was inserted, creating a glass wool-resin-glass wool "sandwich". Several ml of 0.1M sodium acetate, pH 4.9, were forced through the "column" by gentle N$_2$ pressure (1—3 psig), followed by 3 applications each of 190 microliters of 25% acetonitrile (UV grade, Burdick and Jackson, Muskegon, Michigan) and of 190 microliters of 0.1M sodium acetate, pH 4.9. The reaction mixture, previously diluted to 0.6 ml in 0.1M sodium acetate, was passed through the column twice, again under N$_2$ pressure. The column was then washed with 3 applications of 190 microliters H$_2$O. Elution of the probe was achieved by passage of 2×100 microliter aliquots of 25% acetonitrile. The labeled probe was stored in this solvent at −70°C until use, it had 200 K cpm/microliter.

The filters (4 total) prepared as above were washed for 2 hours in 250 ml of 1M NaCl, 50 mM Tris-HCl, pH 8, 1 mM ethylenediamine tetraacetic acid, disodium salt (Na$_2$EDTA), 0.1% sodium dodecyl sulfate (SDS) at 42°C with agitation. They were then "prehybridized" in 100 ml of a solution containing 10X Denhart's solution (Maniatis *et al., supra*, p. 448), 100 microliters/ml denatured salmon sperm DNA (Maniatis *et al., supra*, p. 327) 6X NET (0.9 NaCl, 90 mM Tris-HCl pH 8.3, 6 mM Na$_2$EDTA), 0.1% SDS at 59°C for 1 hour with agitation. The prehybridization mix was decanted and replaced with 20 ml of 6X NET, 0.1% SDS, 250 micrograms/ml *E. coli* t-RNA (Boehringer Mannheim) and 200 microliters of the labeled oligonucleotide. This mixture was kept at 59°C for 2 hours, with constant agitation (Suggs *et al.*, 1981, p. 683 *in*: Developmental Biology Using Purified Genes; D. Brown, editor, Academic Press, New York).

The filters were then washed for 5 minutes at 45°C in 200 ml 6X NET, the solution was decanted and replaced by 200 ml of fresh 6X NET at 45°C and the temperature slowly raised over a twenty minute period to 59°C. The filters were held at 59°C for 5 minutes and the solution was decanted. The damp filters were exposed to XAR film (Kodak, Rochester, new York) at −70°C with a Cronex lightning plus intensifying screen (duPont, Wilmington, Delaware) overnight. Approximately 45 "blotted colonies" retained the hybridized labeled probe while their neighbors did not. Six of the original dark red colonies corresponding to these "hot" blotted colonies were restreaked onto MacConkey plates containing ampicillin (100 micrograms/ml) and incubated overnight at 37°C. The following day a well isolated colony from each of the six streaks was picked and inoculated into 10 ml of L-broth containing ampicillin (100 micrograms/ml) and grown at 37°C for 7 hours.

An inoculum of 2 ml of one culture (designated "A1") was placed into 500 ml of L-broth containing ampicillin (100 micrograms/ml) and incubated overnight at 37°C. The following day, plasmid DNA was extracted from the culture according to the protocol of Birnbaum *et al.*, Nucleic Acid Research 7:1513—1523, 1979.

A sample of 1.5 ml of each of the six cultures was transferred to a microfuge tube and pelleted for 1 minute at 12 K×g in a microfuge. The supernatant liquid was removed and the bacterial pellets were lysed into 1.0 ml of sample buffer for polyacrylamide gel electrophoresis. The buffer contained 2% SDS, 0.1M dithiothreitol, 80 mM Tris-HCl pH 6.8, 10% glycerol and 0.002% bromphenol blue dye. The samples were stored at −70°C overnight. The following day the samples were boiled for 5 minutes and loaded onto an 8.5% polyacrylamide gel prepared according to the procedure described in Heine *et al.*, Journal of Virology *14*:640—651, 1974. After electrophoresis the gels were stained with Coomassie Brilliant Blue according to the protocol of Fairbanks *et al.*, Biochemistry *10*:2606—2617, 1971 and dried onto Whatman 3MM filter paper. This protein analysis confirmed the expression of an approximately 200K dalton fusion protein in five of the six samples analyzed, including the culture designated "A1"). This protein was of similar size to the one expressed in the starting vector.

A sample of 20 micrograms of the plasmid DNA from clone A1 was digested with the restriction endonuclease BstE1 (New England Biolabs) using 5.0 units/microgram of DNA in the restriction buffer described in Example 2. The sample was incubated at 37°C for 90 minutes, at which time it was adjusted to 0.3M sodium acetate and the nucleic acids precipitated by the addition of 2.5 volumes of anhydrous ethanol and incubation for 15 minutes on dry ice. The precipitated DNA was pelleted for 5 minutes at 12K×g at 4°C in a microfuge, rinsed with 70% ethanol and dried under vacuum. The 3' ends of the digested DNA were then labeled with the Klenow fragment of DNA polymerase I (Boehringer Mannheim) using 2 units of enzyme per microgram of DNA, 100 µCi $^{32}$P-dGTP (Amersham 400 Ci/mmol) in a buffer containing 7 mM Tris-HCl pH 7.8, 7 mM MgCl$_2$ and 50 mM NaCl. Incubation was at room temperature (20°C) for 15 minutes. The reaction mixture was adjusted to 2.0M ammonium acetate and the nucleic acids precipitated by the addition of 2.5 volumes of ethanol followed by incubation for 15 minutes on dry ice. The DNA was pelleted for 5 minutes at 12K×g in a microfuge at 4°C, rinsed with 70% ethanol and dried under vacuum. The DNA was resuspended in 0.3M sodium acetate and re-precipitated as described above. The dried DNA pellet was resuspended in deionized distilled water and digested with the restriction endonuclease MstII (New England Biolabs) using 3—4 units of enzyme per microgram of DNA in the restriction buffer described in Example 2. The sample was incubated at 37°C for 2 hours. The mixture then was adjusted to contain 1.2% FICOLL, a nonionic synthetic polymer of sucrose, 10 mM Tris-HCl pH 7.5, 10 mM EDTA, 0.2% bromphenol blue dye, 0.2% xylene cyanol dye, 0.2% amarinth dye, and was electrophoresed through a 2% low-gelling temperature agarose gel (FMC Corporation, Marine Colloids Division, Rockland, Maine) overnight at 30 volts in a buffer containing 40 mM Tris-HCl, 20 mM sodium acetate, 1 mM EDTA at pH 7.2. A radiolabeled fragment about 300 base pairs long was excised from the gel after staining the gel in 1 microgram/ml ethidium bromide in running buffer and visualizing the DNA on a UV transilluminator. The gel slice containing the DNA was placed in a microfuge tube, melted at 65°C and extracted with half a volume of phenol which had been pre-heated to 37°C. The aqueous phase was removed and re-extracted with an equal volume of phenol. The final aqueous phase was removed, 1/10 volume of 5M NaCl added and the sample was centrifuged for 2 minutes at 12K×g in a microfuge. The aqueous phase was removed, the DNA was ethanol precipitated, rinsed and dried as described above. The dried DNA was dissolved in 50 microliters of H$_2$O and was found to contain about 10$^6$ counts per minute. This DNA was then sequenced according to the protocol of Maxim et al. Methods in Enzymology 65: 499—560, 1977. The following nucleotide sequence was obtained:

3'-CCGCCACTAAGTTCCCTAGGTGTGGGCAAGGTGGACGAGCAGATGGAATTGCTAGGGCAGC-5'

The underlined sequence is that of oligonucleotide No. 2 inserted into the starting vector so that the sequence to the left, as written, is part of the EBV-MA coding sequence, and the sequence to the right, as written, is part of the β-galactosidase coding sequence. The entire combined stretch of DNA codes for a fusion protein containing the amino acid sequence recognized by the endopeptidase renin between the EBV-MA and β-galactosidase protein domains.


Example 4
Isolation of EBV-MA-β-galactosidase fusion protein

All work was done at 4°C unless stated otherwise. Cell paste, 9.4 g, from E. coli strain AMA 1004, clone A1, was suspended in 50 mM sodium phosphate, pH 7.1 and 5 mM dithiothreitol to make 100 ml of a 10% suspension (wt/vol). Immediately before cell rupture, 2 ml of 100 mM phenylmethylsulfonyl fluoride (PMSF) in 2-propanol was added to inhibit serine proteases. Cells were ruptured by two passes through a Stanstedt pressure cell at 80 psig, 12 psig back pressure.

The broken cell suspension was clarified at 10,000×g for 30 minutes and to the supernatant liquid (117 ml) was added 16.7 ml of 40% streptomycin sulfate (wt/wt) to precipitate nucleic acids. The mixture was clarified by centrifugation at 10,000×g for 30 minutes.

To 117 ml of the streptomycin sulfate containing supernatant liquid was added 41 ml of 3.8M ammonium sulfate, pH 7 over a period of 30 minutes with stirring. The mixture was stirred for an additional 2 hours and centrifuged at 10,000×g. The supernatant solution was decanted and 30 minutes later the precipitate was dissolved in 20 ml of PBS and dialyzed against 2×200 volumes of PBS for 8 hours each.


Example 5
Cleavage of fusion protein

The product from Example 4 was found to contain 3 mg/ml protein by Lowry analysis. This material was diluted in PBS to a concentration of 362 micrograms/ml.

Purified renin, 456 micrograms/ml was assayed for activity (Poe et al., J. Biol. Chem. 258: 2209—2216, 1983) and found, at a 1:1,000,000 dilution to be able to convert 36 ng/ml/hour of angiotensinogen to angiotensin I. This enzyme was diluted to 3.62 micrograms/ml in PBS. The fusion protein was incubated with 10 microliters of renin at 3.62 micrograms/ml for 4 hours at 37°C to cleave the β-galactosidase protein from the EBV-MA fusion product. Cleavage of the fusion protein to give the above products was confirmed by polyacrylamide gel electrophoresis (Laemmli, Nature *227*:680, 1971), silver staining the gels (Morrissey, Anal. Biochem. *117*:307—310, 1981), and immunoblotting the gels (Burnette, Anal. Biochem. *112*:195—203, 1981) using EBV hyperimmune human serum.

The EBV-MA was purified away from the β-galactosidase by thiogalactoside affinity column chromatography (Germino *et al.*, PNAS *80*:6848—6852, 1983) or by hydrophobic interaction chromatography on phenyl Sepharose® (Pharmacia, Uppsala, Sweden). The phenyl Sepharose® column was equilibrated with PBS, the renin cleaved fusion protein was chromatographed on the column and the EBV-MA was recovered in the unadsorbed fraction.

**Claims**

1. A stretcht of DNA coding for a fusion protein which comprises nucleotides coding for a protein of interest and nucleotides coding for a stably expressed protein, the stretch of DNA having a DNA oligonucleotide which is inserted between the nucleotides coding for the protein of interest and the nucleotides coding for the stably expressed protein, said DNA oligonucleotide, coding for an amino acid sequence cleavable by the endopeptidase, renin, said amino acid sequence being:

X-Pro-A-B-C-D-E-F-Y

wherein

X is His, Ile-His, Tyr-Ile-His, Val-Tyr-Ile-His, Arg-Val-Tyr-Ile-His, Asn-Arg-Val-Tyr-Ile-His, or $(Pro)_n$-His where n is an integer of from 1 to 5,

A is Phe, Trp, Tyr or Leu,

B is His, Leu, Tyr, Ala, Ser or Lys,

C is Leu or Phe,

D is Leu, Ala or Val,

E is Val, Ile, Leu or Ala,

F is Tyr, Phe, Trp, Leu or His, and

Y is absent or is Ser.

2. A vector containing a stretch of DNA coding for a fusion protein according to claim 1 wherein the DNA oligonucleotide is inserted between the nucleotides coding for the protein of interest and the nucleotides coding for the stably expressed protein.

3. A vector according to claim 2 wherein the DNA oligonucleotide is inserted at the precise junction of the nucleotides coding for the protein of interest and the nucleotides coding for the stably expressed protein.

4. A method of preparing a fusion protein cleavable by renin comprising transcribing and translating a stretch of DNA according to claim 1.

5. A procaryotic or eucaryotic host containing any one of the vectors of claims 2—4.

6. A fusion protein prepared by the method according to claim 4.

**Patentansprüche**

1. DNA-Strang, der für ein Fusionsprotein codiert und Nukleotide, die für ein interessierendes Protein codieren, und Nukleotide, die für ein stabil exprimiertes Protein codieren, umfasst, wobei der DNA-Strang ein DNA-Oligonukleotid aufweist, das zwischen die für das interessierende Protein codierenden Nukleotide und die für das stabil exprimierte Protein codierenden Nukleotide insertiert ist, welches DNA-Oligonukleotid für eine durch die Endopeptidase Renin spaltbare Aminosäuresequenz codiert und welche Aminosäuresequenz die Formel aufweist:

X-Pro-A-B-C-D-E-F-Y

worin

X His, Ile-His, Tyr-Ile-His, Bal-Tyr-Ile-His, Arg-Val-Tyr-Ile-His, Asn-Arg-Val-Tyr-Ile-His oder $(Pro)_n$-His ist, worin n eine ganze Zahl von 1 bis 5 ist,

A Phe, Trp, Tyr oder Leu ist,

B His, Leu, Tyr, Ala, Ser oder Lys ist,

C Leu oder Phe ist,

D Leu, Ala oder Val ist,

E Val, Ile, Leu oder Ala ist,

F Tyr, Phe, Trp, Leu oder His ist, und

Y fehlt oder Ser ist.

2. Vektor, der einen DNA-Strang enthält, welcher für ein Fusionsprotein nach Anspruch 1 codiert, worin das DNA-Oligonukleotid zwsichen die für das interessierende Protein codierenden Nukleotide und die für das stabil experimierte Protein codierenden Nukleotide insertiert ist.

3. Vektor nach Anspruch 2, worin das DNA-Oligonukleotid an der genauen Verbindung der für das interessierende Protein codierenden Nukleotide und der für das stabil exprimierte Protein codierenden Nukleotide insertiert ist.

4. Verfahren zur Herstellung eines durch Renin spaltbaren Fusionsproteins, welches des Transkribieren und Translatieren eines DNA-Strangs nach Anspruch 1 umfasst.

5. Prokariotischer oder eukariotischer Wirt, der einen der Vektoren der Ansprüche 2 bis 3 enthält.

6. Fusionsprotein, hergestellt nach dem Verfahren nach Anspruch 4.

**Revendications**

1. Segment d'ADN codant pour une protéine de fusion, qui comprend des nucléotides codant pour une protéine désirée et des nucléotides codant pour un protéine exprimée de manière stable, le segment d'ADN ayant un oligonucléotide de type ADN qui est inséré entre les nucléotides codant pour la protéine désirée et les nucléotides codant pour la protéine exprimée de manière stable, ledit oligonucléotide de type ADN codant pour une séquence d'amino-acides clivable par l'endopeptidase rénine, ladite séquence d'amino-acides étant:

$$X\text{-}Pro\text{-}A\text{-}B\text{-}C\text{-}D\text{-}E\text{-}F\text{-}Y$$

dans laquelle

X est His, Ile-His, Tyr-Ile-His, Val-Tyr-Ile-His, Arg-Val-Tyr-Ile-His, Asn-Arg-Val-Tyr-Ile-His ou (Pro)n-His où n est un entier de 1 à 5,

A est Phe, Trp, Tyr ou Leu,

B est His, Leu, Tyr, Ala, Ser ou Lys,

C est Leu ou Phe,

D est Leu, Ala ou Val,

E est Val, Ile, Leu ou Ala,

F est Tyr, Phe, Trp, Leu on His, et

Y est absent ou est Ser.

2. Vecteur contenant un segment d'ADN codant pour une protéine de fusion selon la revendication 1, dans lequel l'oligonucléotide de type ADN est inséré entre les nucléotides codant pour la protéine désirée et les nucléotides codant pour la protéine exprimée de manière stable.

3. Vecteur selon la revendication 2, dans lequel l'oligonucléotide de type ADN est inséré à la jonction précise des nucléotides codant pour la protéine désirée et des nucléotides codant pour la protéine exprimée de manière stable.

4. Procédé pour la préparation d'une protéine de fusion clivable par la renine comprenant la transcription et la traduction d'un segment d'ADN selon la revendication 1.

5. Hôte procaryote ou eucaryote contenant l'un quelconque des vecteurs des revendications 2 à 3.

6. Protéine de fusion préparée selon le procédé de la revendication 4.